# EUROPEAN PATENT SPECIFICATION

(11) **EP 2 893 919 B1**
(45) Date of publication and mention of the grant of the patent: **12.07.2017**
(21) Application number: 14000064.7
(22) Date of filing: 09.01.2014
(51) Int. Cl.: A61K 9/14, A61K 31/5377

(54) **Formulation of aprepitant with enhanced solubility**
Aprepitant-Formulierung mit erhöhter Löslichkeit
Formulation de l'aprépitant avec solubilité élevée

(43) Date of publication of application: 15.07.2015
(73) Proprietor: SANOFI, 75008 Paris (FR); Zentiva, k.s., 102 37 Praha 10 (CZ)
(72) Inventor: Mathieu, Amandine, 75012 Paris (FR); Greco, Stephanie, 75018 Paris (FR); Nakach, Mostafa, 94320 Thiais (FR); Bardet, Lionel, 91120 Palaiseau (FR); Authelin, Jean Rene, 91180 Saint Germain les Arpajon (FR); Skaria, Cyrus Victor, 403 202 Bambolim, Goa (IN); Beranek, Josef, 160 00 Praha 6 (CZ); Dammer, Ondrej, 253 01 Hostivice (CZ); Krejcik, Lukas, 190 17 Praha-Vinor (CZ); Chalupova, Pavla, 100 00 Praha 10 (CZ); Dumicic, Aleksandra, 10 000 Zagreb (HR)
(74) Representative: Jirotkova, Ivana

(56) References cited:
- WO-A1-2010/149183
- WO-A1-2011/158053

## Description

### Background

Aprepitant (5-([(2R,3S)-2-((R)-1-[3,5-bis(trifluoromethyl)phenyl]ethoxy)-3-(4-fluorophenyl) morpholino]methyl)- 1H-1,2,4-triazol-3(2H)-one) is an antiemetic chemical compound that belongs to a class of drugs called substance P antagonists (SPA) and is used for prevention of chemotherapy-induced nausea and vomiting. This drug substance is a BCS class II due to its poor solubility in water (< 10 µg/mL from pH range 2 to 10) and its moderate permeability (Caco-2 permeability reported at 7.85×10⁻⁶ cm/s). It thus exhibits slow absorption, limited mainly by its solubility resulting in very low oral bioavailability.

Aprepitant is commercially available under the brand name EMEND as capsules containing 40 mg, 80 mg, or 125 mg of aprepitant for oral administration. In spite of EMEND being formulated as a nanoparticulate composition with an average particle size of less than about 1000 nm, the bioavailability is only about 60 - 65% when administered orally.

International Application WO 03/049718 discloses nanoparticulate compositions comprising Aprepitant particles stabilised by at least one surface stabiliser adsorbed onto the surface of the particles, so that an effective average particle size of less than 1000 nm is maintained. A generally described process for preparing such nanoparticulate compositions comprises steps of slurry preparation, pre-milling, media milling, coating dispersion preparation, Wurster column coating, sieving, blending, and encapsulation.

Because of such complexity of the nanoparticle approach, there have been made several attempts to increase the solubility of aprepitant by another ways.

International Application WO 2007/016582 discloses amorphous coprecipitates formed by removing of solvent from a solution of aprepitant and a carrier. Specific examples are given where polyvinylpyrrolidon K-30 is used as the carrier and dichloromethane or dichlormethan in combination with methanol are used as the solvents in all the examples. In all cases, the removal of the solvent was performed using Buchi Rotavapor apparatus. Using this technique, a person skilled in the art would inherently expect high content of the residual solvent in the resulting coprecipitate.

International Application WO 2007/147160 relates to powder compositions of aprepitant with improved solubility properties. The "powder compositions" are defined as a powder of aprepitant itself or a composition of aprepitant along with other excipients in the form of coprecipitates, premixes, solid dispersions, admixtures with surfactants and/or cyclodextrins, and aprepitant particles along with emulsifiers and wetting agents. Coprecipitates of aprepitant with polyethylene glycol were in the form of solid dispersions; no results on solubility, purity or stability of such coprecipitates are shown. Coprecipitate of aprepitant with polyvinylpyrrolidon K-30 was prepared. Solubility of this coprecipitate in water (25°C) was shown to be 0.001 mg/ml.

International Application WO2008/110534 discloses a method showing how to control the dissolution rate of a poorly soluble drug by particle size of inert pellets in the composition.

International Application WO2009/108828 solves the problem of aprepitant solubility by formation of an inclusion complex of aprepitant with at least one cyclodextrin or cyclodextrin derivative.

International Application WO2010/149183 discloses a method of preparation of an aprepitant intermediate composition, which is in the form of solid solution consisting of aprepitant and a matrix material, by extruding the mixture of aprepitant and the matrix material.

Aprepitant is commercialized by Merck & Co. under the brand name Emend and is formulated as a suspension of nanocrystals.

The preparation of a nanoparticulate composition is complicated as it utilizes specialized equipment and involves processing over extended periods of time, making the product uneconomical to manufacture on a large scale. Such particle size reduction process could also result in physical and chemical instability of aprepitant. Furthermore, nanoparticulate products are subject to agglomeration requiring special precautions such as addition of surface stabilizers during the processing and layering directly onto substrates to overcome these problems. To overcome the attractive interparticle forces an intensive and long screening has to be performed in order to select the adequate wetting/dispersant agents. High energetic milling process is required to achieve particles size in the nanometer range. Additional processing step is required in order to get the product in the form of granulates (spray drying or fluid bed granulation). This step will required a second intensive screening in order to select the adequate re-dispersing agent.

It would therefore be highly desirable to develop a pharmaceutical composition of aprepitant that has appreciable solubility and is potentially bioequivalent to the commercial product EMEND but is easy to manufacture and is cost effective. As it is shown in this document, formulation comprising hydroxypropylmethyl cellulose and solid solution of aprepitant with the co-polymer of polyvinyl caprolactam with polyvinyl acetate and polyethylene glycol proved to solve this problem.

The prior art methods suitable for preparation of solid solutions comprise hot melt extrusion, spray drying, lyophilisation and supercritical fluid drying. As the lyophilisation and supercritical fluid drying are not commonly used in the pharmaceutical industry due to labour and cost-ineffective process requiring high investments into special equipment, the spray-drying and hot-melt extrusion are the common methods known in the art for solid solution preparation.

However, even the processes of holt-melt extrusion and spray-drying can cause problems in standard production.

Hot-melt extrusion requires melting of polymer at high temperature and dissolution of aprepitant in the melted solution. This process is limited by thermal degradation of some sensible compounds; on the other hand, if no sufficiently high temperature is used, undesirable recrystallization of the active ingredient can occur which results in a decrease in solubility and thus reduces the absorption and therefore bioavailability. Furthermore, the extruded material needs to be processed generally by cryo-milling in order to get the suitable particles size. This brings additional quite complex process step into the production. Generally, the hot-melt extrusion process is very expensive, not only due to the high investment costs but due to very high operating costs as well caused by high melting temperatures used.

The other typical method for preparation of amorphous solid solutions is using spray drying of an organic solution containing the active ingredient and a polymer(s) and other excipients if necessary.

However spray drying exhibits following disadvantages:
- It is an expensive technique, especially when manipulating organic solvents,
- It typically only works with diluted solutions (<1% or more likely <10% solid content) due to visco-elasticity of polymer solutions, which results in a productivity limitation and a high solvent consumption
- The spray dried polymeric particles are hollow, which results in a low bulk density
- Small scale (e.g. lab scale glas spray dryers or smallest size of pilots, with diameter below 1m) does not allow manufacturing large particles. Therefore there is a systematic quality difference between samples manufactured in small or medium scale units and that performed in large scale equipment, which is a clear disadvantage for pharmaceutical development.
- The spray dried polymeric particles are usually very fine (D90 below 20 µm; bigger particles results in recrystalisation of the active ingredient and thus solid dispersion formation) which results in bad flowability and thus poor processability.

Although the methods of hot-melt extrusion and spray-drying could be used for preparation of the aprepitant solid solutions, there is still a need to provide a fast, robust, cost effective and straightforward process for preparation of the aprepitant solid solution of desired process properties, such as stability, dissolution profiles, particle size and flowability.

WO 2011/158053 discloses a stable nanostructured Aprepitant compositions, comprising nanostructured Aprepitant stabilized by a polymer, in which nanoparticles are distributed. Accordingly, in physical terms, WO 2011/158053 describes a two-phase system.

Therefore, one object of this invention is a process for preparation of the aprepitant solid solution with high aprepitant loading by a continuous drum drying process. Another object of this invention is a pharmaceutical formulation comprising solid solution of aprepitant with the co-polymer of polyvinyl caprolactam with polyvinyl acetate and polyethylene glycol and hydroxypropylmethyl cellulose or poloxamer which exhibits a satisfactory dissolution profile potentially bioequivalent to the commercial product EMEND.

### Summary

The object of this invention is a pharmaceutical formulation wherein it consists of a/ the solid solution of aprepitant in the co-polymer of polyvinyl caprolactam with polyvinyl acetate and polyethylene glycol and b/ the low viscosity grade HPMC, wherein the weight ratio between aprepitant, the co-polymer of polyvinyl caprolactam with polyvinyl acetate and polyethylene glycol and the HPMC is 17:67:16, filled into a hard capsule.

In another embodiment of the invention, the pharmaceutical composition is dry granulated before filling into hard capsules.

### Brief description of the Figures

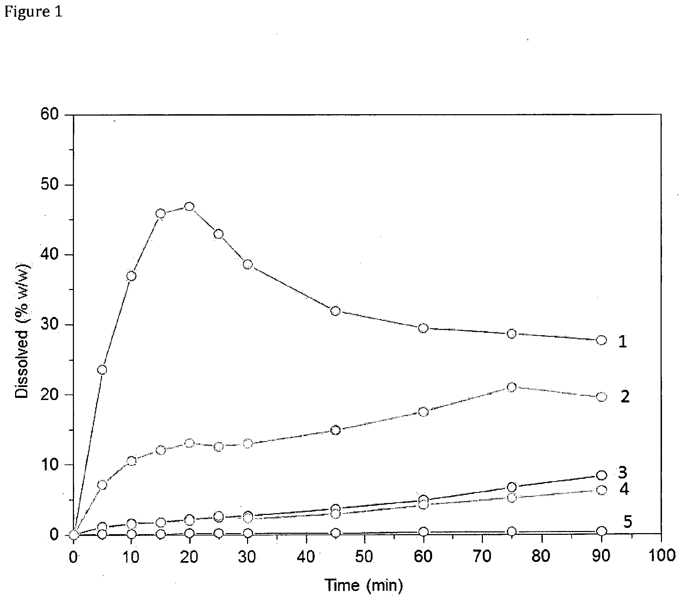

Figure 1: Dissolution profile of aprepitant solid solutions (1 - solid solution of aprepitant and Soluplus, 20:80 by weight; 2 - solid solution of aprepitant and Soluplus, 30:70 by weight; 3 - solid solution of aprepitant and PVP K-30, 20:80 by weight; 4 - solid solution of aprepitant and PVP K-30, 30:70 by weight; 5 - solid solution of aprepitant and Eudragit EPO, 20:80 by weight)

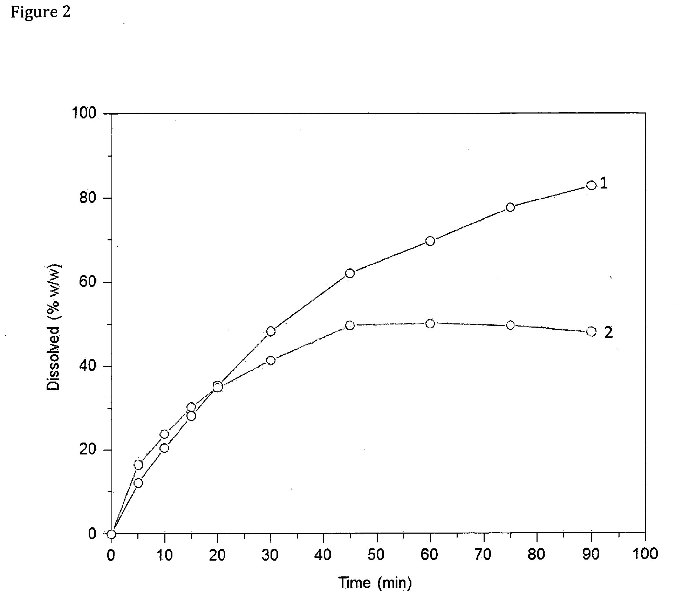

Figure 2: Dissolution profile of compositions of aprepitant solid solutions with auxiliary agent (1 - composition solid solution of aprepitant and Soluplus, 20:80 by weight corresponding to 125 mg aprepitant with 120 mg of HPMC E5; 2 - composition solid solution of aprepitant and Soluplus, 20:80 by weight corresponding to 125 mg aprepitant with 120 mg of poloxamer 237)

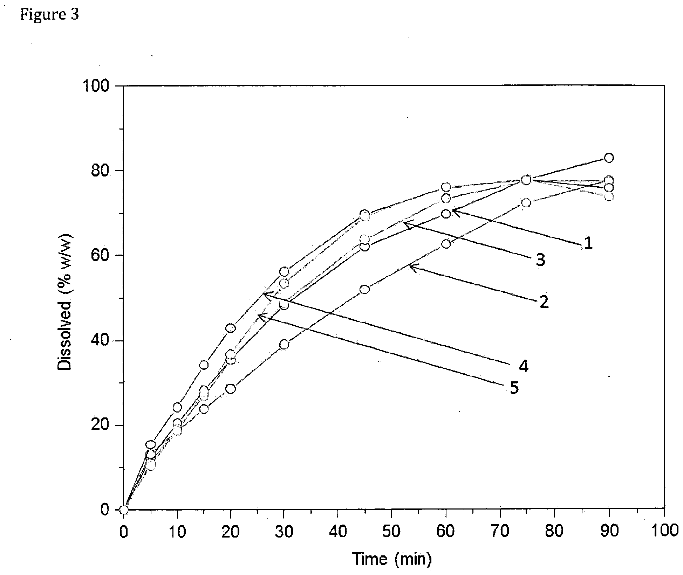

Figure 3: Dissolution profiles of hard capsules comprising dry granulated solid solution of aprepitant and Soluplus, 20:80 by weight corresponding to 125 mg with different levels of HPMC E5 (1-120 mg HPMC E5, 2 - 80 mg HPMC E5, 3 - 60 mg HPMC E5, 4, -40 mg HPMC E5, 5- 20 mg HPMC E5)

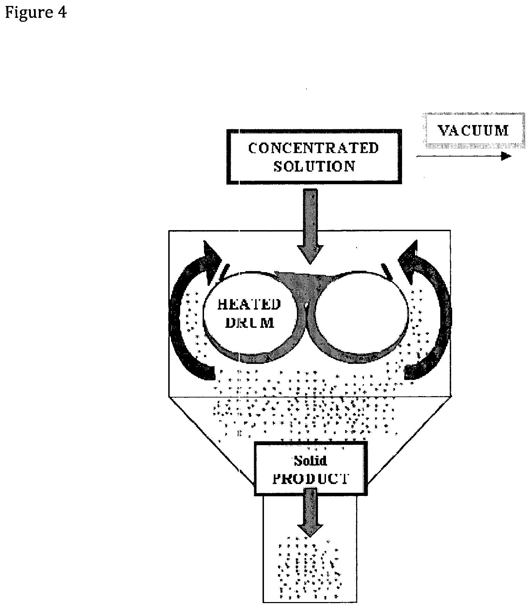

Figure 4: Schematic illustration of the drum-drying process.

### Detailed Description

Aprepitant is an active ingredient with negligible solubility in water media. Its bioavailability is thus limited and it is desirable to increase the solubility of the active ingredient as much as possible. Transformation of the active ingredient into an amorphous solid solution can solve this problem. The solid solutions of aprepitant can be prepared by hot melt extrusion, spray drying, lyophilisation and supercritical fluid drying. The most common methods are the hot melt extrusion and spray drying. However, as it is shown in this document, very advantageous is preparation of the aprepitant solid solutions by a method of drum drying (roller drying).

### Aprepitant solid solutions

For the purpose of this application, by the term of "amorphous solid solution of aprepitant" or "aprepitant solid solution" is meant a solid solution of aprepitant in a polymer wherein the molecules of aprepitant are homogenously distributed within the molecules of the polymer; i.e. the "amorphous solid solution" of aprepitant is a state corresponding to a molecular dispersion of aprepitant within the polymer. For proving the state of solid solution, the glass-transition temperature (Tg) measurement was performed. The melting process with only one Tg indicates that only one phase (in the physic-chemical meaning) is present in the sample, i.e. that the material is in the form of solid solution (e.g. solid dispersion would show at least two Tg peaks).

In international Applications WO 2007/016582 and WO 2007/147160, the solid solutions of aprepitant with polyvinyl pyrrolidone (PVP) were prepared. However, surprisingly, when such prior art was reproduced, the prepared solid solutions were poorly soluble and the dissolution profiles were unacceptable (as shown in Example 1). Therefore the solid solution presented in the prior art is not applicable in the pharmaceutical formulations and in the pharmaceutical industry. For this reason a comprehensive screening of polymers potentially usable in the aprepitant solid solution preparation was performed.

Several polymers were tested in order to prepare solid solutions of aprepitant, such as co-polymer of polyvinyl caprolactam with polyvinyl acetate and polyethylene glycol (Soluplus®); copolymer based on dimethylaminoethyl methacrylate, butyl methacrylate, and methyl methacrylate (Eudragit E PO); copolymer based on methacrylic acic and methyl methacrylate (Eudragit S 100); Hypromellose Accetate Succinate (HPMCAS); Cellulose Acetate Phthalate (CAP); polymer Carboxymethyl Ethyl Cellulose (CMEC); and their combinations, such as co-polymer of polyvinyl caprolactam with polyvinyl acetate and polyethylene glycol (Soluplus®) with HPMCAS; co-polymer of polyvinyl caprolactam with polyvinyl acetate and polyethylene glycol (Soluplus®) with CAP; co-polymer of polyvinyl caprolactam with polyvinyl acetate and polyethylene glycol (Soluplus®) with CMEC; co-polymer of polyvinyl caprolactam with polyvinyl acetate and polyethylene glycol (Soluplus®) with copolymer based on methacrylic acic and methyl methacrylate (Eudragit S 100); polyvinyl pyrrolidone K-30 with CAP; and polyvinyl pyrrolidone with CMEC.

Out of these polymers, the combination of co-polymer of polyvinyl caprolactam with polyvinyl acetate and polyethylene glycol (Soluplus®) with copolymer based on methacrylic acic and methyl methacrylate (Eudragit S 100); and polyvinyl pyrrolidone K-30 with CAP were not suitable for preparation of the aprepitant solid solution (see Example 1). The use of combination of co-polymer of polyvinyl caprolactam with polyvinyl acetate and polyethylene glycol (Soluplus®) with CMEC resulted in the solid dispersion of aprepitant within the polymeric matrix, as indicated by two Tg temperatures (glass-transition temperatures) in the Example 1. The form of solid dispersion is, however, not favorable because the presence of aprepitant undissolved particles causes decrease in solubility in comparison to a homogenous solid solution without any aprepitant particles.

Thus, polyvinyl pyrrolidone, co-polymer of polyvinyl caprolactam with polyvinyl acetate and polyethylene glycol (Soluplus®); copolymer based on dimethylaminoethyl methacrylate, butyl methacrylate, and methyl methacrylate (Eudragit E PO); copolymer based on methacrylic acic and methyl methacrylate (Eudragit S 100); Hypromellose Accetate Succinate (HPMCAS); Cellulose Acetate Phthalate (CAP); polymer Carboxymethyl Ethyl Cellulose (CMEC); and their combinations, such as co-polymer of polyvinyl caprolactam with polyvinyl acetate and polyethylene glycol (Soluplus®) with HPMCAS; co-polymer of polyvinyl caprolactam with polyvinyl acetate and polyethylene glycol (Soluplus®) with CAP; and polyvinyl pyrrolidone with CMEC allowed forming the aprepitant solid solutions.

Surprisingly, the dissolution profiles were unexpectedly low for most of the aprepitant solid solutions (Figure 1), including the prior art aprepitant solid solutions with PVP, making them inapplicable for further usage in the pharmaceutical formulations. Only the aprepitant solid solution with co-polymer of polyvinyl caprolactam with polyvinyl acetate and polyethylene glycol (Soluplus®) provided satisfactory dissolution profile. The dissolution profile of aprepitant solid solution with co-polymer of polyvinyl caprolactam with polyvinyl acetate and polyethylene glycol (Soluplus®) figured in the Figure 1 shows that this aprepitant solid solution can be dissolved in such extent that a state of supersaturation of the dissolution medium by the dissolved aprepitant is possible.

One aspect of this disclosure is therefore aprepitant solid solution with co-polymer of polyvinyl caprolactam with polyvinyl acetate and polyethylene glycol (Soluplus®).

In another aspect of this disclosure is aprepitant solid solution with co-polymer of polyvinyl caprolactam with polyvinyl acetate and polyethylene glycol (Soluplus®) wherein the weight ratio between aprepitant and the co-polymer of polyvinyl caprolactam with polyvinyl acetate and polyethylene glycol (Soluplus®) is between between 1:4 and 3:7.

The preferred aprepitant solid solutions are:
- Aprepitant solid solution with co-polymer of polyvinyl caprolactam with polyvinyl acetate and polyethylene glycol (Soluplus®) in the weight ratio 1:4 (aprepitant: co-polymer)
- Aprepitant solid solution with co-polymer of polyvinyl caprolactam with polyvinyl acetate and polyethylene glycol (Soluplus®) in the weight ratio 3:7 (aprepitant: co-polymer)

The most preferred aprepitant solid solution is the aprepitant solid solution with co-polymer of polyvinyl caprolactam with polyvinyl acetate and polyethylene glycol (Soluplus®) in the weight ratio 1:4 (aprepitant: co-polymer).

These aprepitant solid solutions with the co-polymer of polyvinyl caprolactam with polyvinyl acetate and polyethylene glycol (Soluplus®) provides outstanding dissolution profiles and are stable over long period of time at stress conditions (60°C/0% relative humidity; 30°C / 57% relative humidity; 30°C / 75% relative humidity; 50°C / 57% relative humidity; 50°C / 75% relative humidity).

The aprepitant solid solutions according to the present invention, i.e. aprepitant solid solution with co-polymer of polyvinyl caprolactam with polyvinyl acetate and polyethylene glycol (Soluplus®) preferably with the weight ratio of the active ingredient to the polymer, used in the compositions according to the present invention, can be prepared by hot melt extrusion, spray drying, lyophilisation and supercritical fluid drying. However, the advantageous method of preparation of the aprepitant solid solutions according to the present invention is the method of drum drying (roller drying). In aspect of this invention, the preferred aprepitant solid solutions described above are prepared by the method of drum drying.

### Preparation of aprepitant solid solutions by method of drum-drying

One of the aspects of this invention is a new, effective and robust process of preparation of the aprepitant solid solutions by a continuous drum drying process. This process enables preparation of solid solutions using lower amount of solvents in comparison with spray-drying and using lower temperatures and less energy in comparison with the hot-melt extrusion.

Drum dryers are typically used to isolate food products and more rarely they are used for isolation of pharmaceutical ingredients, such as macrocyclic antibiotics, polysaccharides and enzymes, mostly in an amorphous state as these kinds of compounds do not crystallize. This method, however, has never been used for preparation of an amorphous solid solution of an active pharmaceutical ingredient in a polymer, such solid solution being designed to be an intermediate for a drug product production.

Surprisingly it has been found that the process of continuous drum drying is suitable for preparation of amorphous solid solutions of aprepitant which are chemically as well as physico-chemically stable over long period of time even under hard stress storage conditions.

Therefore, the continuous drum drying process offers an alternative to spray drying process. The similarity in both techniques is that they both offer a fast drying of a solution of aprepitant and a polymer, which does not give enough time for the crystallization of the active ingredient. However, from a process point of view, the processes are fundamentally different. As shown here below, the continuous drum drying process is advantageous over the spray-drying:
- A concentrated solution of polymer, aprepitant and optionally other excipients can be used (up to 50% weight solid content, i.e. up to 9 times less solvent per kg of solid in comparison to spray-drying)
- The method is much more productive: a 100 l (0.2 m²) pilot plant for drum drying process versus a 2 m³ device for spray-drying
- The process is highly flexible in term of batch sizes, i.e. it can be used independently of the volumes produced. This is advantageous as well for the development process because
- The quality of the product (i.e. the aprepitant solid solution) does not change at a scale-up from the development batch sizes to the production batch sizes.
- The high speed of the process in combination with low volumes of the used solvents brings lowering of the production costs.

Furthermore, the particle size of the aprepitant solid solutions prepared by the invented method can be affected by the setup of the grid size in the calibration step *d*/, as described below. Thus the particle size of the aprepitant solid solutions prepared by the drum drying method can be set up directly to a particle size suitable for using in the pharmaceutical formulations, without requiring additional step of decreasing the particle size by conventional methods, e.g. milling. Preferably, the aprepitant solid solutions were prepared with a particle size distribution characterized by the D50 value of (i.e. 50% w/w of the particles is smaller than) 50-60 µm (measured by laser scattering) which corresponded to usage of a 630 µm grid. The particle size of the produced aprepitant solid can be, however, optionally further decreased by conventionally used methods.

The aprepitant solid solutions prepared by the drum drying exhibit surprisingly good flowability in comparison to spray-drying where the particles are usually too fine and very poorly flowable. The bulk density of the aprepitant solid solutions prepared by the drum drying is 0.2-0.4 g/ml.

### Drum drying principle

The drum drying process converts a liquid in a powder in one step. A scheme of a double drum drying equipment is illustrated in Fig. 4. The process consists of using a double drum drier with counter rotating drums placed in stainless cabinet which could be operated under negative or atmospheric pressure. The typical separation distance between the 2 drums is 0.1 to 1 mm. The solution is distributed on the hot drums and the solvent evaporates during the partial rotation of the drums. As the process is carried out usually at a lower pressure than the atmospheric pressure and as the drum is heated, the thin layer will dry very quickly. After approximately a ¾ turn, the dry product is removed by a scraper. A monocylinder machine can be used identically and it would provide the same results.

The continuous process of drum drying for production of aprepitant solid solution comprises following steps:
a/ preparation of a concentrated solution of aprepitant, a polymer and optionally other excipients:
   - The concentration of the solid content is up to 30% by weight
   - % of aprepitant in the solution is 6 to 9 % by weight. The weight ratio between aprepitant and the polymer (or polymers) is to be between 1:4 and 3:7.
   - The solvent for the preparation of the solution is a volatile solvent or a combination of volatile solvents selected from the group of methylene chloride, acetone and other ketone, methanol, ethanol and others alcohols, and in a general manner all organic solvents with a boiling point ≤100°C (water is included). ICH (International Conference on Harmonization of Technical Requirements for Registration of Pharmaceuticals for Human Use) class III and class II solvents are preferable from a toxicological point of view, but other solvents can be used, provided the product can be dried under ICH requirements. The preferred solvents are ethanol and acetone or their mixture. The most preferred is the solvent mixture ethanol and acetone in the ratio 40/60 (w/w).
b/drum drying of the solution prepared in the step a/ (continuous drying of the solution prepared in the step a/ by distribution of the solution on a heated surface in a thin layer):
   - The solution prepared in the step a/ is distributed on the rotating drum, so that the drum is coated with a thin layer of the concentrated solution.
   - The pressure in the machine is equal to the atmospheric pressure or lower than the atmospheric pressure. The atmospheric pressure is understood as a pressure of 1013.25 mbar. Preferably, the pressure is lower than the atmospheric pressure. More preferably the pressure is lower than 100 mbar. Most preferably the pressure is lower than 50 mbar.
   - The temperature of the drum is to be set 1-20°C above the boiling temperature (at the considered pressure) of the solution prepared in the step a/. For the most preferred the solvent mixture ethanol and acetone in the ratio 40/60 (w/w) is the temperature of the drum set at 65-75°C, preferably at 70°C.
   - The drum rotation is usually in the range 1 to 10 rpm in order to allow the duration of the step b/ (i.e. a residence time of the material on the surface of the drum) between 6 s and 1 minute. This corresponds to a solution flow rate of 5-10l/h.
   - The layer thickness of the solution at the beginning of the step b/, given by the distance between the drums, can be 50 - 300 µm, preferably 50-200 µm and most preferably it is about 100 µm. The value "about 100 µm" is to be understood as 80-120 µm.
   - The process of the step b/ can be performed in one layer in a mono-cylinder machine or in two layers in a double-cylinder machine.
c/ removal of the dried product from the drum surface (removal of the dried product from the heated surface)
   - The solid product in the form of aprepitant solid solution is removed from the drum surface by a knife (see Fig. XX)
d) calibration of the particle size of the drum dried product using oscillatory mill equipped with a grid size of 300-1500 µm, preferably 630 µm
e) secondary drying using static oven under vacuum pressure at 45°C followed by 60°C

### Pharmaceutical formulations comprising the aprepitant solid solution

The pharmaceutical compositions according to the present invention and described above, consisting of the aprepitant solid solution with co-polymer of polyvinyl caprolactam with polyvinyl acetate and polyethylene glycol (Soluplus®) admixed with poloxamer or with the low viscosity grade HPMC, can be used in preparation of the pharmaceutical formulations.

Accordingly, another aspect of the presented invention is a pharmaceutical formulation comprising the pharmaceutical composition according to the present invention, consisting of the aprepitant solid solution with co-polymer of polyvinyl caprolactam with polyvinyl acetate and polyethylene glycol (Soluplus®) admixed with poloxamer or with the low viscosity grade HPMC, and optionally other pharmaceutically acceptable excipients, e.g. fillers, disintegrants, binders and/or lubricants. Preferably, the pharmaceutical formulations are in a form intended for oral administration, i.e. in the form of tablets or capsules, or other final drug forms for oral administration known from the state of the art. The most preferred are the oral hard capsules.

The pharmaceutical formulations according to this invention comprise
- an amount corresponding to the aprepitant dose of 50 - 200 mg, typically 10-70% (w/w) of the pharmaceutical composition consisting of a/ the aprepitant solid solution with the co-polymer of polyvinyl caprolactam with polyvinyl acetate and polyethylene glycol (Soluplus®) and b/ poloxamer or with the low viscosity grade HPMC
- optionally one or more fillers, e.g. anhydrous lactose, lactose monohydrate, microcrystalline cellulose, silicified microcrystalline cellulose, anhydrous calcium hydrogen phosphate, starch, pregelatinized starch, mannitol, sorbitol and other fillers known form the art, typically in the amount of 30-80% (w/w)
- optionally one or more disintegrants, e.g. sodium croscarmelose, crospovidone, starch, hydroxypropyl cellulose low-substituted, colloidal silica, sodium carboxymethyl starch, and other disintegrants known form the art, typically in the amount up to 10% (w/w)
- optionally one or more binders, e.g. polyvinylpyrrolidones" copovidone, hydroxypropyl cellulose, and/or hydroxyethyl cellulose, hydroxypropylmethyl cellulose and other binders known form the art, typically in the amount up to 10% (w/w)
- optionally one or more glidants, e.g. colloidal silica, magnesium carbonate, stearic acid or its metallic salts such as magnesium stearate, sodium stearyl fumarate, magnesium palmitate, magnesium oleate, hydrogenated vegetable oil, hydrogenated castor oil, talc, polyethylene glycols, and other glidants known form the art, typically in the amount up to 5% (w/w)
- optionally one or more lubricants, e.g. sodium stearyl fumarate, stearic acid, stearic acid salts such as magnesium stearate, calcium stearate, magnesium palmitat, magnesium oleate, hydrogenated vegetable oil, hydrogenated castor oil, talc, colloidal anhydrous silica, macrogols (polyethylene glycoles), and any mixtures thereof, typically in the amount up to 5% (w/w).

The particle size of the aprepitant solid solutions should be such that the D90 value is between 3 and 1500 µm, preferably 5-750 µm and more preferably 50-250 µm. The D90 value means that at least 90% of the particles are smaller than the given value.

The preferred pharmaceutical formulation according to the present invention is in the form of oral hard capsule. The capsule shell can be based on gelatin, HPMC, carrageenan, modified starch or any material commonly used and known from the art. The hard capsule can be filled with the pharmaceutical composition consisting of the aprepitant solid solution according to the present invention and poloxamer or the low viscosity grade HPMC; and optionally with other pharmaceutically acceptable excipients (e.g. fillers, glidants, disintegrants).

The capsule filling can be in the form of blended powders or the ingredients can be dry granulated (e.g. by roller compaction) prior the filling into the capsules.

In the preferred embodiment, the pharmaceutical formulation according to the present invention is in the form of oral hard capsule filled with the pharmaceutical composition consisting of the aprepitant solid solution according to the present invention and poloxamer.

In another preferred embodiment, the pharmaceutical formulation according to the present invention is in the form of oral hard capsule filled with the pharmaceutical composition consisting of the aprepitant solid solution according to the present invention and the low viscosity grade HPMC.

Thus, the pharmaceutical formulation according to the present invention consists of the aprepitant solid solution with co-polymer of polyvinyl caprolactam with polyvinyl acetate and polyethylene glycol (Soluplus®) admixed with the low viscosity grade HPMC, wherein the weight ratio between aprepitant, the co-polymer of polyvinyl caprolactam with polyvinyl acetate and polyethylene glycol (Soluplus®) and the low viscosity grade HPMC is 17:67:16. The most preferred is the HPMC of the grade E5.

### Methods

### Analysis - DSC (Differential Scanning Calorimetry)

DSC measurements were performed on a Perkin Elmer Pyris 1 DSC.
A MDSC Q200 supplied by TA Instruments was used for the Tg measurements. Samples from 2 to 10 mg were submitted to a modulated heating program with a mean rate of 3°C/min, a period of 60s and amplitude of 1°C, from ambient temperature to 250°C.

### X-ray diffraction

The analyses were performed using a Bruker D8 diffractometer with a Bragg-Brentano focusing geometry (*θ*-*2θ*) in the reflection mode. A tube with an anticathode of copper (40kV / 40mA) supplies an incident radiation that is filtered by iron to eliminate the K*_{β}* radiation. The beam is collimated both by Soller slits and by fixed slits before the detector which limit diffusion. The parameters employed are as follows: scan range from 11 to 34 degrees 2*θ*, with a step of 0.02º(2θ) and a rate of one step per second.

### Dissolution

900 ml of phosphate buffer, pH 6.8 with 0.1% (by volume) SDS addition, baskets with glass Watman filter, 100 rpm was used.

### HPLC analysis

column: Kinetex C18, 2,6um, 30 x 4,6 mm (Pheneomenex)
mobile phase: MF A : MF B : MF C = 35% : 60% : 5%
(MF A: 1 ml H₃PO₄ to 1000 ml water, MF B: acetonitrile, MF C: methanol flow rate: 1.0 ml/min column temperature: 30°C

### Examples

### Example 1 - preparation of aprepitant solid solution

Aprepitant and the polymer (or polymer mixture) were solubilized at a room temperature in the solvent mixture ethanol / acetone (40/60 w/w) with a total solid concentration comprised between 5 and 10%. The solution was spray-dried with solution flow rate 12 ml/min with inlet temperature 100°C and outlet temperature 70-75°C. The product of the spray-drying was dried at 75°C until a constant weight was reached.

The solid solutions were prepared using the polymers and ratios shown in the table 1.
The amorphous state of the produced material was confirmed by a XRPD measurement. The physic-chemical state of the material was confirmed to be a solid solution by the DSC analysis. The melting process with one glass-transition temperature proved that the material was in the state of the solid solution. In the case of the combination of Soluplus with CMEC, two Tg temperatures were observed, meaning that the aprepitant was not homogenously distributed within the polymer, i.e. that in some extent the aprepitant recrystalised back into particles and the material was in the form of solid dispersion.

**Table 1**

| | **Concentration in the solid state** | | | **Product** | | | |
|---|---|---|---|---|---|---|---|
| **N° Batch** | **Polymer type** | **(% w/w)** | | **Quality** | **Amorphous (XRPD)** | **Nr of Tg** | **Tg (°C)** |
| | | **Polymer** | **API*** | | | | |
| HAS316 | PVP K-30 | 80 | 20 | OK | OK | 1 | 149 |
| HAS317 | Soluplus | 80 | 20 | OK | OK | 1 | 72 |
| HAS318 | Eudragit S100 | 80 | 20 | cotton like | OK | 1 | 149 |
| ZYR051 | Eudragit EPO | 80 | 20 | OK | OK | 1 | 60 |
| ZYR052 | PVP K-30 | 70 | 30 | OK | OK | 1 | 138 |
| ZYR053 | Soluplus | 70 | 30 | OK | OK | 1 | 80 |
| HAS319 | Eudragit EPO | 70 | 30 | OK | OK | 1 | 69 |
| HAS320 | Soluplus | 40 | 20 | very electrostatic | OK | 1 | 95 |
| | HPMCAS | 40 | | | | | |
| HAS321 | Soluplus | 40 | 20 | OK | OK | 1 | 119 |
| | CAP | 40 | | | | | |
| HAS322 | Soluplus | 40 | 20 | OK | OK | 2 | 95, 133 |
| | CMEC | 40 | | | | | |
| HAS323 | Soluplus | 40 | 20 | gelling of the solution | NA*** | NA | NA |
| | Eudragit S100 | 40 | | | | | |
| HAS324 | PVP K-30 | 40 | 20 | biphasic gelling solution | NA | NA | NA |
| | CAP | 40 | | | | | |
| HAS325 | PVP K-30 | 40 | 20 | OK | OK | 1 | 125 |
| | CMEC | 40 | | | | | |

| | | | | | | | |
|---|---|---|---|---|---|---|---|
| *API = aprepitant, **Solid = total solid conc. in the solution prior drying, ***NA = not applicable | | | | | | | |

### Example 2 - dissolution of the aprepitant solid solution

The dissolution profiles of aprepitant solid solutions were determined. The results are shown in the Figure 1. The aprepitant solid solution with Eudragit EPO was not soluble at all. The solubility of the aprepitant solid solution with PVP K-30 was very low (only about 5% of aprepitant was dissolved within 90 min). The dissolution profiles of the other prepared solid solutions were very low as well, similar or lower than the aprepitant solid solution with PVP K-30 (not shown). The aprepitant solid solutions with Soluplus® showed the best solubilities. The aprepitant solid solution with Soluplus® in the ratio of aprepitant to Soluplus® 3:7 enabled dissolution of 13% of aprepitant within 20 min and aprepitant solid solution with Soluplus® in the ratio of aprepitant to Soluplus® 1:4 enabled dissolution 46% of aprepitant within 20 min. The fast onset of the dissolution of aprepitant solid solution itself is fundamental for reaching the bioavailability of the aprepitant, as the kinetic of absorption of the dissolved amount of aprepitant is very fast in the human body. After 20 min of measurement there was observed a decrease in the dissolved amount of aprepitant caused by reverse precipitation of aprepitant from the supersaturated solution. However, the possibility to achieve the supersaturated state of the dissolution medium by release of aprepitant from the solid solution with Soluplus® proved that this solid solution is suitable for development of a pharmaceutical formulation with a desirable bioavailability of aprepitant.

### Example 3 - stability of the aprepitant solid solutions with Soluplus®

Amorphous solid solutions of aprepitant with Soluplus® were obtained by drum-drying method using a double drum dryer. Aprepitant and Soluplus® were solubilized at a room temperature in the solvent mixture ethanol / acetone (40/60 w/w) with a total solid concentration comprised 20% (w/w) or 30% (w/w), respectively, the ratio of aprepitant to Soluplus® was 3:7 and 1:4, respectively. The drum (roller) temperature was 70°C, the pressure was 22 mbar and the solution flow rate was 10 liters per hour. The distance between the drums was setup to 200 µm to provide two layers of the dried solution of 100 µm. The grid size was 630 µm. The products of the drum-drying were analyzed by XRPD and DSC and amorphous solid solutions of aprepitant were proved. The particle size of the prepared aprepitant solid solutions was 52 µm (D90, laser scattering).

The aprepitant solid solutions with Soluplus® were subjected to a stability testing. The results are shown in the Table 2. The stability was evaluated by X-ray diffraction and by the DSC analyses. The aprepitant solid solutions proved to be stable for at least 65 days under all the tested stress conditions.

**Table 2**

| **N° Batch** | **Ratio*** | **Amorphous solid solution for** | | |
|---|---|---|---|---|
| | | **60°C / 0% RH** | **30°C/57% RH** | **50°C/37% RH** |
| HAS0317 | 20:80 | >119Days | >78Days | >110Days |
| ZYR053 | 30:70 | >71Days | >70Days | >65Days |

| | | | | |
|---|---|---|---|---|
| *Ratio = ratio of aprepitant to Soluplus® (w/w) | | | | |

### Example 4 - composition comprising aprepitant solid solution with Soluplus®

The aprepitant solid solution with Soluplus® in the ratio 1:4 prepared by the drum drying method described in the Example 3 (D90 58 µm, laser scattering) was used for preparation of a pharmaceutical composition. Aprepitant solid solution (corresponding to 125 mg of aprepitant) was blended with 120 mg of HPMC E5 in a weight ratio aprepitant: Soluplus® : HPMC being 17 : 67 : 16.

Similarly, the aprepitant solid solution (corresponding to 125 mg of aprepitant) was blended with 120 mg of poloxamer 237 in a weight ratio aprepitant: Soluplus® : poloxamer being 17 : 67 : 16.

Dissolution profiles of these pharmaceutical compositions were measured (Figure 2). The pharmaceutical composition of aprepitant solid solution with poloxamer 237 enabled supersaturation of the solution by 50% (by weight) of aprepitant. The supersaturated state was maintained over 90 min of the measurement, no reverse precipitation of dissolved aprepitant was observed. The pharmaceutical composition of aprepitant solid solution with HPMC enhanced the dissolved amount of aprepitant even more; more than 80% (by weight) of aprepitant was dissolved within the 90 min measurement, no reverse precipitation of dissolved aprepitant was observed.

### Example 5 - formulations comprising aprepitant solid solution with Soluplus® and HPMC

Pharmaceutical formulations comprising aprepitant solid solution with Soluplus® in the form of hard capsules were prepared. Aprepitant solid solution with Soluplus® in the ratio 1:4 (in an amount corresponding to 125 mg of aprepitant) was blended with 20, 40, 60, 80 and 120 mg of HPMC. The powder blend was roller compacted and the granules were filled into hard gelatin capsules. Dissolution profiles of these capsules were determined (Figure 3).

In all cases, HPMC enhanced both supersaturated concentration of aprepitant in the solution and duration of the supersaturation. The extent of HPMC effect depended on its concentration. The lowest amount of HPMC (20 mg) enhanced the supersaturation stage as much as 60 mg. The high concentration of HPMC (80-120 mg) maintains supersaturated stage for long time.

## Claims

1. A pharmaceutical formulation, **characterized in that** it consists of the solid solution of aprepitant in the co-polymer of polyvinyl caprolactam with polyvinyl acetate and polyethylene glycol and the low viscosity grade HPMC, wherein the weight ratio between aprepitant, the co-polymer of polyvinyl caprolactam with polyvinyl acetate and polyethylene glycol and the HPMC is 17:67:16, filled into a hard capsule.

2. The pharmaceutical formulation according to the claim 1, **characterized in that** the solid solution of aprepitant in the co-polymer of polyvinyl caprolactam with polyvinyl acetate and polyethylene glycol and the low viscosity grade HPMC are dry granulated before filling into the capsule.

3. A process of preparation of the aprepitant solid solution contained in the pharmaceutical formulation according to the claim 1 by the method of drum drying.

4. The process according to the claim 3, **characterized in that** it comprises the following steps a/-e/:
a/ preparation of a concentrated solution of aprepitant and the polymer
b/ continuous drying of the solution prepared in the step a/ by distribution of the solution on a heated surface of the rotating drum in a thin layer, so that the drum is coated with a thin layer of the concentrated solution, wherein the temperature of the rotating drum is set 1-20°C above the boiling temperature of the solution prepared in the step a/ and wherein the speed of the drum rotation is in the range 1 to 10 rpm and the flow rate of the solution is 5-10 l/h
c/ removal of the dried product from the heated surface of the rotating drum
d) calibration of the particle size of the drum dried product using oscillatory mill equipped with a grid size of 300-1500 µm
e) optionally, secondary drying until reaching the constant weight of the product.

5. The process according to the claim 4, **characterized in that** the concentration of aprepitant in the solution prepared in the step a/ is 6 to 9 % (w/w)

6. The process according to the claim 4 or 5, **characterized in that** the solvent used in the step a/ is selected from the group consisting of water, methylene chloride, acetone, methanol, ethanol, propanol, isopropanol and their mixtures.

7. The process according to the claim 5, **characterized in that** the solvent is the mixture ethanol and acetone in the ratio 40/60 (w/w).

8. The process according to any of the claims 4-7, **characterized in that** the step b/ is performed at the atmospheric pressure or at a pressure lower than the atmospheric pressure.

9. The process according to the claim 8, **characterized in that** the step b/ is performed at a pressure lower than 100 mbar.

10. The process according to the claim 9, **characterized in that** the pressure is lower than 50 mbar.

11. The process according to any of the claims 4-10, **characterized in that** the calibration in the step d/ is performed by using the oscillatory mill equipped with 630 µm grid size.

12. The process according to any of the claims 4-11, **characterized in that** the optional secondary drying in the step e/ is performed under vacuum pressure at 45°C-60°C.

13. The process according to the claims 4-12, **characterized in that** it consists of the steps a/-e/:
a/ preparation of a concentrated solution of aprepitant and the polymer which is the co-polymer of polyvinyl caprolactam with polyvinyl acetate and polyethylene glycol,
- the concentration of aprepitant in the solution is 6 to 9 % (w/w)
- the solvent is the mixture of ethanol and acetone in the ratio 40/60 (w/w).
b/ continuous drying of the solution prepared in the step a/ by distribution of the solution on a heated surface of the rotating drum in a thin layer, so that the drum is coated with a thin layer of the concentrated solution,
- wherein the temperature of the rotating drum is set to 70°C, and
- the pressure is 22 mbar, and
- the flow rate of the solution is 10 l/h
c/ removal of the dried product from the heated surface of the rotating drum
d) calibration of the particle size of the drum dried product using oscillatory mill equipped with a grid size of 630 µm
e) optionally, secondary drying until reaching the constant weight of the product performed under vacuum pressure at 45°C-60°C.

## Patentansprüche

1. Pharmazeutische Formulierung, **dadurch gekennzeichnet, dass** sie aus einer festen Lösung von Aprepitant in dem Copolymer von Polyvinylcaprolactam mit Polyvinylacetat und Polyethylenglycol sowie HPMC mit niedrigem Viskositätsgrad besteht, wobei das Gewichtsverhältnis von Aprepitant zum Copolymer von Polyvinylcaprolactam mit Polyvinylacetat und Polyethylenglycol zu der HPMC 17:67:16 beträgt, abgefüllt in einer Hartkapsel.

2. Pharmazeutische Formulierung nach Anspruch 1, **dadurch gekennzeichnet, dass** die feste Lösung von Aprepitant in dem Copolymer von Polyvinylcaprolactam mit Polyvinylacetat und Polyethylenglycol und die HPMC mit niedrigem Viskositätsgrad vor dem Abfüllen in die Kapsel trocken granuliert werden.

3. Verfahren zur Herstellung der festen, in der pharmazeutischen Formulierung nach Anspruch 1 enthaltenen Aprepitant-Lösung durch das Verfahren der Walzentrocknung.

4. Verfahren nach Anspruch 3, **dadurch gekennzeichnet, dass** es die folgenden Schritte a/-e/ umfasst:
a/ Herstellung einer konzentrierten Lösung von Aprepitant und dem Polymer,
b/ kontinuierliche Trocknung der in der Schritt a/ hergestellten Lösung durch Verteilen der Lösung auf einer erwärmten Oberfläche der sich drehenden Walze in einer dünnen Schicht, so dass die Walze mit einer dünnen Schicht der konzentrierten Lösung beschichtet wird, wobei die Temperatur der sich drehenden Walze auf 1-20 °C über der Siedetemperatur der in Schritt a/ hergestellten Lösung eingestellt ist und wobei die Geschwindigkeit der Rotation der Walze in dem Bereich von 1 bis 10 Umdrehungen pro Minute liegt und die Durchflussmenge der Lösung 5-10 l/h beträgt,
c/ Entfernung des getrockneten Produktes von der erwärmten Oberfläche der sich drehenden Walze,
d) Kalibrierung der Partikelgröße des walzengetrockneten Produkts unter Verwendung einer Schwingmühle, die mit einer Siebgröße von 300-1500 µm ausgestattet ist,
e) wahlweise eine sekundäre Trocknung, bis die Gewichtskonstanz des Produktes erreicht ist.

5. Verfahren nach Anspruch 4, **dadurch gekennzeichnet, dass** die Konzentration von Aprepitant in der in Schritt a/ hergestellten Lösung 6 bis 9 % (w/w) beträgt.

6. Verfahren nach Anspruch 4 oder 5, **dadurch gekennzeichnet, dass** das in Schritt a/ verwendete Lösungsmittel aus der Gruppe ausgewählt ist, die aus Wasser, Methylenchlorid, Aceton, Methanol, Ethanol, Propanol, Isopropanol und Mischungen davon besteht.

7. Verfahren nach Anspruch 5, **dadurch gekennzeichnet, dass** es sich bei dem Lösungsmittel um ein Gemisch von Ethanol und Aceton im Verhältnis 40/60 (w/w) handelt.

8. Verfahren nach einem der Ansprüche 4-7, **dadurch gekennzeichnet, dass** der Schritt b/ bei Atmosphärendruck oder bei einem Druck unterhalb des Atmosphärendrucks durchgeführt wird.

9. Verfahren nach Anspruch 8, **dadurch gekennzeichnet, dass** Schritt b/ bei einem Druck unterhalb von 100 mbar durchgeführt wird.

10. Verfahren nach Anspruch 9, **dadurch gekennzeichnet, dass** der Druck weniger als 50 mbar beträgt.

11. Verfahren nach einem der Ansprüche 4-10, **dadurch gekennzeichnet, dass** die Kalibrierung in Schritt d/ unter Verwendung der Schwingmühle mit einer Siebgröße von 630 µm durchgeführt wird.

12. Verfahren nach einem der Ansprüche 4-11, **dadurch gekennzeichnet, dass** die wahlweise sekundäre Trocknung in Schritt e/ bei 45 °C-60 °C unter Vakuumdruck durchgeführt wird.

13. Verfahren nach den Ansprüchen 4-12, **dadurch gekennzeichnet, dass** es aus den Schritten a/-e/ besteht:
a/ Herstellung einer konzentrierten Lösung von Aprepitant und des Polymers, welches das Copolymer von Polyvinylcaprolactam mit Polyvinylacetat und Polyethylenglycol ist,
- die Konzentration von Aprepitant in der Lösung beträgt 6 bis 9 % (w/w)
- das Lösungsmittel ist ein Gemisch aus Ethanol und Aceton im Verhältnis 40/60 (w/w).
b/ kontinuierliche Trocknung der in Schritt a/ hergestellten Lösung durch Verteilen der Lösung auf einer erwärmten Oberfläche der sich drehenden Walze in einer dünnen Schicht, so dass die Walze mit einer dünnen Schicht der konzentrierten Lösung beschichtet wird,
- wobei die Temperatur der sich drehenden Walze auf 70 °C eingestellt ist und
- der Druck 22 mbar beträgt und
- die Durchflussmenge der Lösung 10 l/h beträgt,
c/ Entfernung des getrockneten Produktes von der erwärmten Oberfläche der sich drehenden Walze,
d) Kalibrierung der Partikelgröße des walzengetrockneten Produkts unter Verwendung einer Schwingmühle mit einer Siebgröße von 630 µm,
e) wahlweise eine sekundäre Trocknung bei 45 °C-60 °C unter Vakuumdruck, bis die Gewichtskonstanz des Produktes erreicht ist.

## Revendications

1. Une formulation pharmaceutique **caractérisée en ce qu'**elle consiste en la solution solide d'aprépitant dans le copolymère de polyvinyl caprolactame avec de l'acétate de polyvinyle et du polyéthylène glycol et la HPMC à faible viscosité, dans laquelle le rapport pondéral entre l'aprépitant, le polymère de polyvinyl caprolactame avec l'acétate de polyvinyle et le polyéthylèneglycol et le HPMC est de 17 :67 :16, introduit dans une capsule dure.

2. La formulation pharmaceutique selon la revendication 1, **caractérisée en ce que** la solution solide d'aprépitant dans le copolymère de polyvinyl caprolactame avec l'acétate de polyvinyle et le polyéthylène glycol et la HPMC à faible viscosité sont granulées à sec avant leur remplissage dans la capsule.

3. Un procédé de préparation de la solution solide d'aprépitant contenue dans la formulation pharmaceutique selon la revendication 1, par le procédé de séchage par tambour.

4. Le procédé selon la revendication 3, **caractérisé en ce qu'**il comprend les étapes a) à e) suivantes:
a) préparation d'une solution concentrée d'aprépitant et du polymère
b) séchage continu de la solution préparée dans l'étape a) par distribution de la solution sur une surface chauffée d'un tambour rotatif en une couche mince, de sorte que le tambour soit revêtu d'une couche mince de la solution concentrée, dans lequel la température du tambour rotatif est réglée de 1 à 20°C au-dessus de la température d'ébullition de la solution préparée dans l'étape a) et dans laquelle la vitesse de rotation du tambour est comprise entre 1 et 10 tr/min et le débit de la solution est de 5 à 10 l/h
c) élimination du produit séché de la surface chauffée du tambour rotatif
d) calibrage de la taille de particule du produit séché par tambour en utilisant un broyeur oscillant équipé d'une grille dont les mailles sont de 300 à 1500 µm
e) éventuellement, séchage secondaire jusqu'à atteindre le poids constant du produit.

5. Le procédé selon la revendication 4, **caractérisé en ce que** la concentration d'aprépitant dans la solution préparée dans l'étape a) est de 6 à 9% (p/p)

6. Le procédé selon la revendication 4 ou 5, **caractérisé en ce que** le solvant utilisé dans l'étape a) est choisi dans le groupe consistant en l'eau, le chlorure de méthylène, l'acétone, le méthanol, l'éthanol, le propanol, l'isopropanol et leurs mélanges.

7. Le procédé selon la revendication 5, **caractérisé en ce que** le solvant est le mélange d'éthanol et d'acétone dans le rapport 40/60 (p/p).

8. Le procédé selon l'une quelconque des revendications 4 à 7, **caractérisé en ce que** l'étape b) est effectuée à la pression atmosphérique ou à une pression inférieure à la pression atmosphérique.

9. Le procédé selon la revendication 8, **caractérisé en ce que** l'étape b) est effectuée à une pression inférieure à 100 mbar.

10. Le procédé selon la revendication 9, **caractérisé en ce que** la pression est inférieure à 50 mbar.

11. Le procédé selon l'une quelconque des revendications 4 à 10, **caractérisé en ce que** le calibrage dans l'étape d) est effectué en utilisant le broyeur oscillant équipé d'une grille dont les mailles sont de 630 µm.

12. Le procédé selon l'une quelconque des revendications 4 à 11, **caractérisé en ce que** le séchage secondaire optionnel de l'étape e) est effectué sous pression sous vide à 45°C-60°C.

13. Le procédé selon les revendications 4 à 12, **caractérisé en ce qu'**il comprend les étapes a) à e) suivantes:
a) préparation d'une solution concentrée d'aprépitant et du polymère qui est le copolymère de polyvinyl caprolactame avec de l'acétate de polyvinyle et du polyéthylèneglycol,
- la concentration d'aprépitant dans la solution est de 6 à 9% (p/p)
- le solvant est le mélange d'éthanol et d'acétone dans le rapport 40/60 (p/p).
b) séchage continu de la solution préparée dans l'étape a) par distribution de la solution sur une surface chauffée du tambour rotatif en une couche mince, de sorte que le tambour soit revêtu d'une couche mince de la solution concentrée, dans lequel
- la température du tambour rotatif est réglée à 70°C, et
- la pression est de 22 mbar, et
- le débit de la solution est de 10 l/h
c) élimination du produit séché de la surface chauffée du tambour rotatif
d) calibrage de la dimension des particules du produit séché par tambour en utilisant un broyeur oscillant équipé d'une grille dont les mailles sont de 630 µm
e) éventuellement séchage secondaire jusqu'à atteindre le poids constant du produit sous pression sous vide à 45°C-60°C.
